# EUROPEAN PATENT SPECIFICATION

(11) **EP 3 276 515 B1**
(45) Date of publication and mention of the grant of the patent: **04.09.2024**
(21) Application number: 17180140.0
(22) Date of filing: 06.07.2017
(51) Int. Cl.: G16H 40/63, G16H 40/67, G16H 40/20, A61F 5/48, G08B 21/20, A61F 13/42

(54) **ENURESIS ALERTS, AMELIORATION, AND TRAINING**
ENURESEWARNUNGEN, -VERBESSERUNG UND -TRAINING
ALERTES D'ÉNURÉSIE, AMÉLIORATION ET FORMATION

(30) Priority: 06.07.2016 US 201615202585
(43) Date of publication of application: 31.01.2018
(73) Proprietor: Theos Medical Systems, Inc., Santa Clara, CA 95054 (US)
(72) Inventor: Bhatia, Saket, Santa Clara, California (US); Bhatia, Ankush, Santa Clara, California (US)
(74) Representative: Marks & Clerk LLP

(56) References cited:
- US-A- 4 163 449
- US-A1- 2005 099 294
- US-A1- 2005 242 946
- US-A1- 2008 052 030
- US-A1- 2014 200 538
- US-A1- 2016 120 454
- US-B1- 7 250 547
- ANONYMOUS: "External WiFi Antenna | eBay", 19 April 2014 (2014-04-19), XP055428386, Retrieved from the Internet <URL:https://web.archive.org/web/20140419045741/http://www.ebay.com/bhp/external-wifi-antenna> [retrieved on 20171123]
- ANONYMOUS: "Transceiver - Wikipedia", 6 April 2016 (2016-04-06), XP055428384, Retrieved from the Internet <URL:https://en.wikipedia.org/w/index.php?title=Transceiver&oldid=713852060> [retrieved on 20171123]
- ANONYMOUS: "port - definition of port from YourDictionary.com", 18 April 2008 (2008-04-18), XP055428561, Retrieved from the Internet <URL:https://web.archive.org/web/20080418185350/http://www.yourdictionary.com/port> [retrieved on 20171124]
- ANONYMOUS: "Signal - Wikipedia", 5 June 2016 (2016-06-05), XP055428092, Retrieved from the Internet <URL:https://en.wikipedia.org/w/index.php?title=Signal&oldid=723888490> [retrieved on 20171123]
- MEDPAGE LIMITED T/A EASYLINK UK |: "Bed Wetting Trainer - Adult Incontinence Alarm", 9 May 2015 (2015-05-09), XP055427560, Retrieved from the Internet <URL:https://web.archive.org/web/20150509181638/http://www.easylinkuk.co.uk/page53.html> [retrieved on 20171121]
- DR. SAGIE BEDWETTING CLINICS: "STOPEE(TM) Bedwetting alarm", 21 May 2016 (2016-05-21), XP055427559, Retrieved from the Internet <URL:https://web.archive.org/web/20160521183519/http://www.bedwettingtherapy.com/category/stopee-bedwetting-alarm> [retrieved on 20171121]
- SIMIK MOHAMED Y E ET AL: "Automated Alarm System for Diaper Wet Using GSM", 2014 IEEE 17TH INTERNATIONAL CONFERENCE ON COMPUTATIONAL SCIENCE AND ENGINEERING, IEEE, 19 December 2014 (2014-12-19), pages 1799 - 1803, XP032730287, DOI: 10.1109/CSE.2014.330

## Description

This application describes technologies that can be used with inventions, and other technologies, described in one or more of the following documents. These documents are sometimes referred to herein as the "Included Disclosures," the "Incorporated Documents," or variants thereof.

No aspect of these documents is intended to be limiting in any way. The scope of the invention remains defined by the claims of this Application. These Included Documents are additive in nature, and can be combined with the technical matter described herein, in whole or in part.

Each document description includes, where applicable, its Application Serial Number, Filing Date, and First Inventor:

| Serial No. | Filing Date | First Inventor |
|---|---|---|
| US 8,253,573 | Mar. 25, 2010 | Saket BIIATIA |
| 14/292,949 | June 2, 2014 | Saket BHATIA |
| 14/958,949 | Dec. 4, 2015 | Saket BHATIA |
| 15/045,271 | Feb. 17,2016 | Saket BHATIA |

### 2. Field of the Disclosure

### 2.1. FIELD OF THE DISCLOSURE

This Application generally relates to enuresis amelioration, alerts, and training, and similar issues.

For one example, this Application includes information relating to sensing, alerting and signaling, analysis and reporting, patient care and sensor care, statistical and other review of data relating to alerts and signals, and other sensing or treatment functions; with respect to enuresis, other medical fluids, other electrolytes, and other sensed events. For another example, this Application includes information relating to server communication, sensor identification, telemetry and data backup, system maintenance and customer assistance, business methods, and other system functions; with respect to sensors, receivers of alerts and signals, data relating to alerts and signals, and other system apparatus or methods.

Other and further possibilities are described herein.

### 2.2. RELATED ART

The approaches described in this section could be pursued, but are not necessarily approaches that have been previously conceived. Therefore, unless specifically indicated herein, the approaches described in this section are not prior art to the claims in this application and are not admitted to be prior art by inclusion in this section.

Enuresis (sometimes referred to as "bedwetting") is the event when a patient loses bladder control during sleep, and voids urine into their underclothes, clothes, or bedding. This is sometimes referred to herein as a "wetting event." A caregiver should be available to wake the patient, send them to a restroom, and assist them in replacing any wetted objects, and assist them in returning to sleep. Typically the patient will be a child, in which case the wetting might be an isolated event; however, when wetting events are chronic, the caregiver, who will typically be a parent or guardian, will wish to assist the child in learning to awake in time to arrive at the restroom and avoid a wetting event. The patient could also be an adult, such as a post-menopausal woman, an elderly person, a post-surgical patient, or another person lacking bladder control, for each of whom enuresis might be a relatively temporary or a more extended problem. In such cases, the caregiver might be a hospital nurse, a practical nurse, a volunteer, a close relative, or another person.

To assist the patient in waking quickly, and to assist the caregiver in reaching the patient quickly, the patient and caregiver can use an enuresis alert system. Typically, an alert system includes a sensor and an alerting device. The sensor would detect the wetting event, and trigger the alerting device. The alerting device would, in response to the sensor, present an alert to the patient or to the caregiver. In such cases, the alert system would either wake the patient directly, or would inform the caregiver, so that the caregiver could wake the patient and assist the patient as described. For example, the sensor would include an electrical circuit that is completed by the presence of urine (or other electrolytes), thus providing an electrical trigger to the alert. Also for example, the alerting device would include an audible alarm that sounds when triggered by the sensor. The alarm would either wake the patient directly, or would inform the caregiver that a wetting event has occurred, so that the caregiver can provide patient care.

Enuresis alert systems are typically available in one of two types: wired or wireless. Wired alert systems couple the sensor directly to the alerting device, while wireless alert systems couple the sensor to a wireless transmitter, which sends a wireless signal to a wireless receiver, which is coupled directly to the alerting device. Wired systems have the disadvantage of being uncomfortable for the patient, and the further disadvantage of requiring a relatively loud alarm to inform the caregiver. Wireless systems mostly avoid these disadvantages, but at the cost of having the disadvantage of being substantially more expensive, the further disadvantage of operating in a relatively noisy portion of the EM (electromagnetic) spectrum, and the further disadvantage of draining battery power even when only awaiting their trigger. If noise in the EM spectrum is too great, sensor alerts could be lost, or spurious sensor alerts could occur. If battery power is drained too much, the alerting system could be insufficiently loud, or could fail completely.

One problem with some known enuresis alert systems is that, when a wetting event occurs, urine could cause corrosion with the sensor's contacts, particularly when electrical current continues to be coupled to those contacts. Thus, wetting events could involve the patient or caregiver removing the sensor while the patient is concurrently rapidly attending the restroom. This could be both complex and subject to mistakes. This problem is exacerbated when the enuresis alert systerns includes a wired coupling between the sensor and the alerting device, as the wiring itself can present an obstacle to the patient or caregiver.

Another problem with some known enuresis alert systems is that, when a wetting event occurs and the patient is alerted, it is desirable for the patient to wake quickly, such as to limit the amount of wetting due to the wetting event. While known enuresis alert systems terminate their alarms in response to disconnection by the patient, they do not record the amount of time taken by the patient to wake and disconnect the alarm, nor do they record the progress---if any---by the patient in waking more quickly in response to the alarm. Superior alacrity by the patient has advantages that known enuresis alert systems do not promote.

Another problem with some known enuresis alert systems is that, when a wetting event occurs, only the patient or only the caregiver is alerted; that is, only one, not both, are alerted. With wired systems, only the patient would be alerted, unless the alerting system is so loud that it can be heard several rooms away. With wireless systems, the sensor might be coupled, similar to wired systems, to alert only the patient or to alert only the caregiver. Alternatively, wireless systems could couple the sensor to alert more than one person, but with the drawback that wireless receivers for alerting devices could be relatively expensive.

Another problem with some known enuresis alert systems is that, when a wetting event occurs, the caregiver typically wishes to record the event. However, due to the desire for rapid response and for substantially cleaning effort, it could occur that the wish to record the event is forgotten, or that data with respect to the event are not recorded accurately or precisely. Recording the wetting event could be important: The amount of time that the patient avoids a wetting event is generally considered, for children, to be about 14 consecutive days. Moreover, the record of wetting events could be used for predicting future events, and for avoiding those future events.

Another problem with some known enuresis alert systems is that it can be desirable to train the patient (such as a child) to respond rapidly to a wetting event. Thus, it might be desirable for the child to wake immediately, turn off the alarm, and continue any further urination in a restroom. One measure of progress (such as by the child) in avoiding future wetting events, might include a measure of speed in responding to a wetting event, such as an amount of time the child takes in performing these steps. However, as with recording the event itself, it could occur that the wish to time the speed of response, and the wish to record that timing information, might be forgotten, or that data with respect to the event are not recorded accurately or precisely.

Another problem with some known enuresis alert systems is that it can be difficult for a user of the system to obtain customer service. First, the user's request for customer service would come within ordinary business hours. Second, the user's request would involve contacting a human being at customer service, providing information to identify the user and their equipment, and providing information to identify the problem. Third, customer service might involve technical support, such as in the form of a trouble ticket. The request could involve several days of effort, which could involve restarting the patient's training to avoid wetting events from the beginning.

US 2005/242946 A1 (James E. Hubbard JR., et al.) discloses a patient activity monitoring (PAM) system with customizable alarms indicating a patient's status, including incontinent events. In the event the patient requires immediate assistance, the PAM system alerts an associated pager .

If the primary caregiver assigned to that pager does not respond, the PAM system initiates a secondary page to a back-up caregiver. The document US 2005/242946 A1 does not disclose the alert processor being configured to immediately direct the alert signal to the secondary caregiver without waiting for the primary caregiver to respond, if more than one wetting event is occuring concurrently.

### 2.3. SOME DRAWBACKS OF THE KNOWN ART

Each of these issues, either alone or in combination with others (whether mentioned herein or otherwise), at some times, or in some conditions, can cause one or more problems with respect to enuresis amelioration, alerts, and training, with respect to similar and other matters, or with respect to some combination or conjunction thereof.

### 3. Brief Summary of the Disclosure

This Summary is provided to introduce one or more concepts, in a simplified form, otherwise and further described in this Application. This Summary does not intend to specify particular features of the claimed subject matter, nor does this Summary intend to be used to determine the scope of the claimed subject matter.

### 3.1. THIS APPLICATION

This Application provides patentable subject matter that can ameliorate the problems described above, as well as others.

Aspects and preferred features are set out in the accompanying claim. The scope of the invention is defined by the appended claim 1.

### 3.2. SYSTEM, METHOD, AND DEVICES

There is provided an apparatus including an alert processor, having an input port disposed to receive a signal output from a sensor in response to a fluid exiting a body of a patient, having instructions determining whether the signal output from the sensor indicates a wetting event, and having an output port disposed to send an alert signal in response to the wetting event; an alert presenter including an alarm responsive to the alert signal.

The alarm may be responsive to one or more of: a set of multiple patients subject to wetting events.

The alert processor has access to second instructions, the second instructions directing the alert signal to at least one or more of a set of alert presenters.

Aspects can be combined and it will be readily appreciated that features described in the context of one aspect can be combined with other aspects.

### 33. POSSIBLE APPLICABILITY

After reading this Application, those skilled in the art would recognize that many variations of techniques shown in this Application are possible that would be workable, and would not require undue experiment or further invention. The characteristics and possible utility of the embodiments described in this Application are not all-inclusive.

Those skilled in the art would recognize both additional advantages and additional features beyond those explicitly shown or suggested in this Application.

After reading this Application, those skilled in the art would also recognize that techniques shown in this Application are applicable to more than just the specific embodiments shown herein. The examples described herein are not intended to be limiting in any way. One or more combinations of these examples, in whole or in part, are also possible.

For one example, the system can be configured to detect biological electrolytes other than urine. Some examples include those found in blood, fecal matter, lymph, mucus, pus, saliva, skin oils, spinal fluid, sweat, tears, vomit, and other bodily substances. In one such case, the system can be configured to detect and inform the patient about onset of menses.

For another example, the system can be configured to detect non-biological electrolytes. Some examples include those found in alcohol, juices, water and other drinking materials, and other foods and non-foods.

Other and further possibilities are described herein.

### 3.4. DESCRIBED APPLICATIONS NOT EXCLUSIVE

These examples are not exclusive from other uses. Instead, they are meant to describe one or more possible applications of systems, methods, devices, and other techniques shown in this Application.

### 4. Brief Description of the Figures

In the figures, like references generally indicate similar elements, although this is not strictly required by the text.
Figure 1 shows a conceptual drawing of a system.
Figure 2 shows a conceptual drawing of a clamp.
Figure 3 shows a conceptual drawing of a method.

After reading this Application, those skilled in the art would recognize that the figures are not necessarily (1) drawn to scale for construction, or (2) specify any particular location or order of construction. After reading this Application, those skilled in the art will recognize that many alternatives, in construction, shape, size, and otherwise, would allow them to make and use the invention, without undue experiment or further invention.

### 5. Detailed Description of A Preferred Embodiment

### 5.1. TERMINOLOGY

### 5.1.1. General Terms and Phrases

Ideas and technologies shown or suggested by this Application should be thought of in their most general form, including without limitation, considering one or more of the following:
The phrases and terms "Application," "this Application," "this Disclosure," and variants thereof, generally refer to this Specification, Drawings, Figures, and Claims, all other parts of this Application, and all facts known in the art at the time of filing, and all facts that can be rationally concluded therefrom.

The phrases and terms "disposed," "disposed for," "disposed to," and variants thereof, generally refer to the possibility that a particular element, collection of elements, portion of an element, or linkage between or among elements, is capable of (and optionally, well suited to) performing the described activity.

The phrases and terms "in one example," "in one embodiment," "in one implementation," "in one scenario," "in possible examples," "in possible embodiments," "in possible implementations," "in possible scenario," and variants thereof, generally refer to the possibility that a particular characteristic, feature, or structure, described herein is included in at least one possible embodiment of the invention. Multiple uses of this phrase do not necessarily all refer to the same possible embodiment. Rather, the specific particular characteristic, feature, or structure, described herein might be combined in any suitable manner into one or more distinct possible embodiments.

The phrases and terms "perform," and variants thereof, generally refer (in the context of a program of instructions) any one or more means by which those instructions are executed or interpreted, or a device (such as a computing device) otherwise conducts the process indicated by that program of instructions. A program of instructions can be detected or interpreted at one location, and executed or its process conducted at another location. A program of instructions can be performed by a portion of a device, rather than the entire device, or by one or more devices, or by one or more portions of devices (the same device or different devices). A program of instructions can be performed by an emulated device, such as a virtual machine, "sandbox" environment, or otherwise. A program of instructions can be performed in part, halted or paused or stopped, transferred to another device, in whole or in part, and possibly continued.

The phrases and terms "relatively," and variants thereof, generally refer to any relationship in which a comparison is possible, including without limitation "relatively less," "relatively more," and otherwise. In the context of the invention, where a measure or value is indicated to have a relationship "relatively," that relationship need not be precise, need not be well-defined, and need not be by comparison with any particular or specific other measure or value. For one example, whenever a measure or value is "relatively increased" or "relatively more," that comparison need not be with respect to any known measure or value, but might be with respect to a measure or value held by that measurement or value at another place or time, or with respect to a measure or value commonly used in the art.

The phrases and terms "substantially," and variants thereof, generally refer any circumstance in which a determination, measure, value, or otherwise; is equal, equivalent, nearly equal, nearly equivalent, or approximately; what the measure or value is recited to be. For example, the phrases and terms "substantially all," and variants thereof, generally refer to any circumstance in which all, except possibly a relatively minor amount or number, have the stated property. For example, the phrases and terms "substantially none," and variants thereof, generally refer any circumstance in which none, except possibly a relatively minor amount or number, have the stated property. For example, the phrases and terms "substantial effect," and variants thereof, generally refer any circumstance in which an effect might be detected or determined.

The phrases and terms "techniques," and variants thereof, generally refer to any material suitable for description, including without limitation all such material within the scope of patentable subject matter. Whenever a method step is described, those skilled in the art would know, without further invention or undue experiment, that this application thereby also describes (1) at least a first product, such as one maintaining instructions that are interpretable by a computing device, where those instructions direct one or more devices to perform that method step; and (2) at least a second product, such as one capable of performing that method step.

### 5.1.2. Specific Terms and Phrases

The phrases and terms "alarm," "alert," "signal," and variants thereof, can generally refer to any technique for informing a patient, caregiver, or other person, regarding a wetting event, or recording information relating thereto. For example, an alarm can include a sound (such as a buzzer or noise), a visible signal (such as a flashing screen or other light), a haptic signal (such as shaking or vibration), or another type of detectable information.

The phrases and terms "caregiver," and variants thereof, can generally refer to any person with the task of assisting or caring for a patient, as that term is described herein, in the event of a wetting event. A caregiver can also be assigned the task of waking the patient to prevent a wetting event, dealing with the consequences of a wetting event, or other related tasks.

The phrases and terms "instructions," can generally refer to any information that can be understood by a processor to perform operations. For example, "instructions," can generally refer to directions to perform operations, to data used in those operations, and to other signals that might be used by a processor. When applied thereto, the phrases and terms "execute," "interpret," and variants thereof, generally refer to operation of a processor, in response to those instructions, to perform those operations.

The phrases and terms "patient," and variants thereof, can generally refer to any person for whom enuresis, bedwetting, wetting events, for whom other leakage of fluids is being detected, or related events. More generally, these phrases and terms can generally refer to any person or persons for whom a sensor is to be used to detect an event. For example, a sensor can be used to detect when a person falls asleep or awakens, has their temperature or blood pressure meet selected criteria, or other events of interest.

The phrases and terms "publish," and variants thereof, can generally refer to any event during which information is sent from one entity (such as a first device or person) to another entity (such as a second device or person). For example, an alert or other signal can generally be published by the alert processor to one or more alert presenters, possibly including one or more of: a patient, a primary caregiver, a backup caregiver, a supervisory caregiver, and storage.

The phrases and terms "user" and variants thereof, generally refer any one or more persons, devices, software elements, or groups thereof, authorized or capable of interacting with one or more portions of a system disclosed herein, or authorized or capable of triggering one or more steps of a method disclosed herein. For example, a "user" can include one or more persons; one or more programs disposed to make requests of, and receive responses from, the system; one or more software elements disposed to control the system or method; an artificial intelligence or machine learning technique capable of interacting with the system or method; or combinations or conjunctions thereof.

The phrases and terms "wetting event," and variants thereof, can generally refer to any event in which enuresis, bedwetting, for whom other leakage of fluids, occurs, or any related events. More generally, these phrases and terms can generally refer to any proximate cause or result of any such event, such as any time relaxation of muscles, opening of strictures or wounds, or leakage of fluids occurs, whether or not immediately detected.

### 5.2. FIGURES AND TEXT

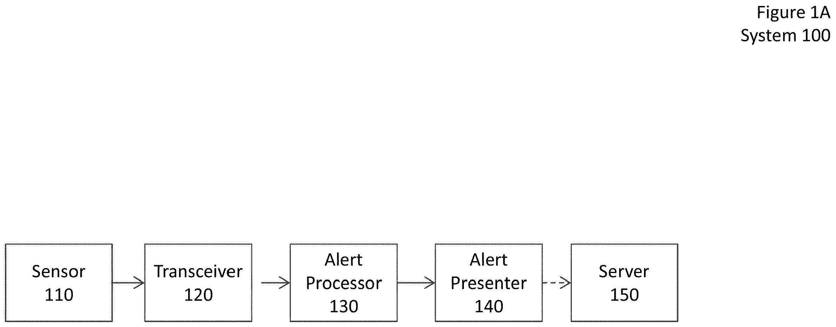

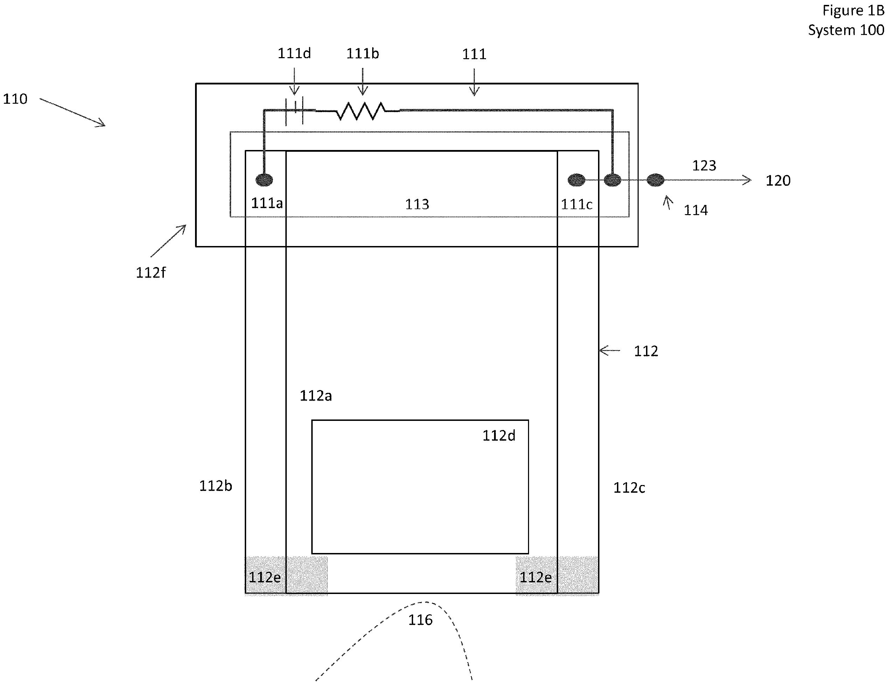

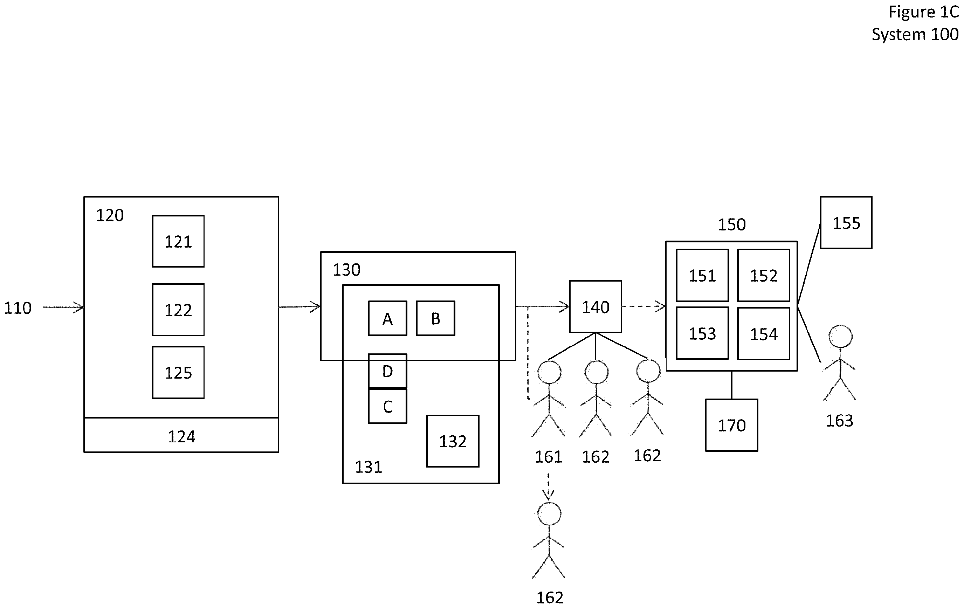

### 5.2.1. Figure 1

Figure 1 shows a conceptual drawing of a system.

A system 100 can include elements shown in the figure, such as at least the following: a sensor 110, a transceiver 120, an alert processor 130, an alert presenter 140, a server 150, and other elements necessary or convenient to give effect to the functions described herein.

In one embodiment, the system 100 is usable by a patient 161, one or more caregivers 162, one or more operators 163, and with a server interface 170.

### Sensor

The sensor 110 can include elements shown in the figure, such as at least the following: an electrical circuit 111, a replaceable test strip 112, an (optional) clamp 113, an output node 114, and a waterproof cover 115. A bodily exit 116 for urine (or another electrolyte) is not part of the sensor 110, but would typically be associated with a patient. Although the test strip 112 is referred to herein as "replaceable," it can be "disposable," in the sense that a used test strip 112 can be disposed of (such as in a trash can or other receptacle, after a wetting event) and a new test strip 112 can be incorporated into the electrical circuit 111 instead of the old test strip 112. In this context, the term "replaceable," and variants thereof, are intended to be more general than the term "disposable," and variants thereof.

The electrical circuit 111 can include a positive node 111a, a resistive element 111b, a negative node 111c, and a power source 111d (such as a battery or a wall plug). In some embodiments, the resistive element 111b can limit the amount of current that flows through the electrical circuit 111 when the electrical circuit 111 is activated.

In this context, the electrical circuit 111 remains uncompleted until the replaceable test strip 112 is present and a wetting event occurs. When the replaceable test strip 112 is present and a wetting event occurs, the electrical circuit 111 becomes completed, including the positive node 111a, the resistive element 111b, the negative node 111c, and the power source 111d.

In other possible implementations, the electrical circuit 111 may omit the resistive element 111b and the power source 111d; these elements may be integrated into the transceiver 120. This can have the similar effect that the electrical circuit 111 is completed, using electrical components that are integrated with the transceiver 120.

The replaceable test strip 112 can include elements shown in the figure, such as at least the following: a wicking element 112a, a first conductive node 112b, a second conductive node 112c, a conductive patch 112d, an adhesive side 112e, and a waterproof covering 112f. In this configuration, the replaceable test strip 112 is optionally held by the clamp 113 (or optionally stuck to the patient's underwear (or other clothing, or a body location) using an adhesive strip, as described herein), with the positive node 111b being coupled to the first conductive node 112b, and with the negative node 111c being coupled to the second conductive node 112c.

The wicking element 112a can be disposed near the bodily exit 116 with the conductive patch 112d proximal thereto, with the effect that when a wetting event occurs, the urine is absorbed by the wicking element 112a, which couples the first conductive node 112b to the second conductive node 112c, thus coupling the positive node 111b to the negative node 111c.

The conductive patch 112d can have the effect that less urine is needed to couple the positive node 111b to the negative node 111c. The urine wets less of the wicking element 112a, thus less time is taken before the positive node 111b couples to the negative node 111c.

The adhesive side 112e can be coupled, such as by a removable glue adhesive, to the patient's clothing. For example, in one context, the adhesive side 112e can be coupled to an outside surface of the patient's underwear, so as to allow wicking of fluid into the replaceable test strip 112 in the even of a wetting event. In this context, the waterproof covering 112f can be disposed between the outside surface of the patient's underwear and any of the patient's further clothing, with the effect of preventing the fluid from the wetting event from wicking further from the replaceable test strip 112 into the patient's further clothing. Of course, if the fluid spreads through the patient's underwear, the fluid might further spread from the outside surface of the patient's underwear to the patient's further clothing or to the patient's bedding.

In one implementation, the adhesive side 112e is all that is necessary to dispose the test strip 112 near to the body exit 116, so the clamp 113 is optional. In an alternative implementation, the test strip 112 can alternatively or in addition be held in place by an (optional) clamp 113, with the effect of preventing the test strip 112 from unwanted movement while the patient (child) is asleep.

When the replaceable test strip 112 is stuck to the patient's underwear (or other clothing, or a body location) using an adhesive strip, the replaceable test strip 112 can be stuck on an inside location (thus, between the bodily exit 116 and the patient's clothing) or on an outside location (thus, near the bodily exit 116 but on the patient's clothing). In either such case, the replaceable test strip 112 is disposed so that the replaceable test strip 112 generally absorbs fluid from the bodily exit 116, with one or more effects noted herein.

The waterproof cover 115 is coupled to the wicking element 112a, with the effect that the wicking element 112a can absorb urine from the bodily exit 116 without wetting the patient's underclothes, clothes, or bedding. As noted above, though, if the fluid spreads through the patient's underwear, the fluid might further spread from the outside surface of the patient's underwear to the patient's further clothing or to the patient's bedding. The waterproof cover 115 can also make it easier to remove the wicking element 112a without the caregiver's hands or the patient's hands becoming dirty from fluid from the wetting event. However, as noted above, if the fluid has spread beyond the wicking element 112a itself, the (optional) waterproof cover 115 might not be very effective in preventing the caregiver's hands or the patient's hands from becoming dirty from fluid from the wetting event.

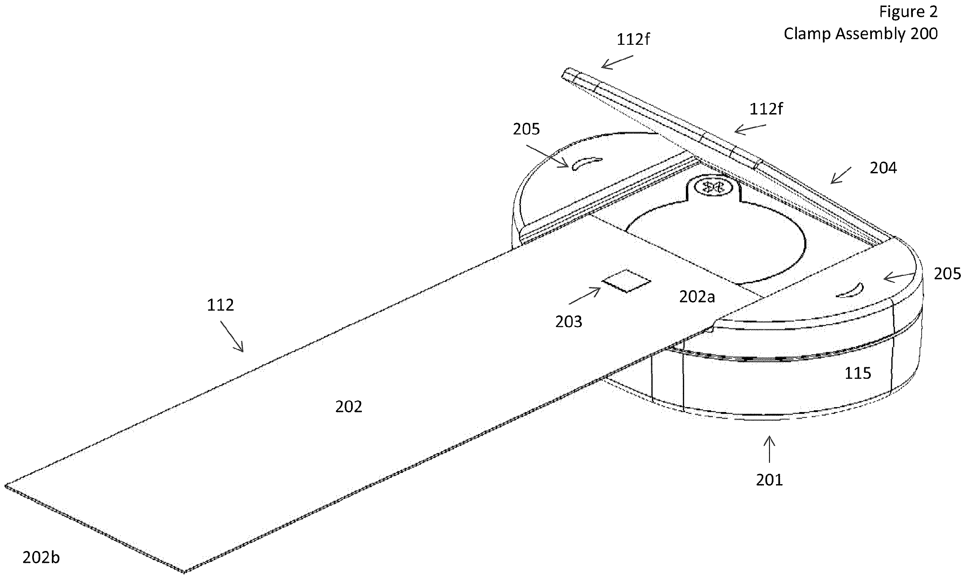

Attachment of the replaceable test strip 112, either to a portion of the sensor 110, to a portion of the transceiver, or to some other element, is described in other and further detail with respect to figure 2.

As described herein, the replaceable test strip 112 in the sensor 110 can both assist in detecting a wetting event, and can be replaced after the event in preparation for a possible new event. Thus, the sensor 110 can be maintained without extensive cleaning, instead by involving only removing the test strip 112 from the sensor 110 after a wetting event, and replacing the replaceable test strip 112 in the sensor 110.

### Transceiver

The transceiver 120 can be coupled to the sensor 110, and can receive a signal from the sensor 110 indicative of a wetting event. For example, the wetting event can be (1) not in progress, that is, there is no current wetting event involving the sensor 110; (2) in progress, that is, the wetting event is ongoing and urine is being vented into the sensor 110; or (3) finished, that is, the wetting event has occurred and urine has been completely vented onto the sensor 110.

In other embodiments, the transceiver 120 can include at least some of the components of the electrical circuit 111, with the effect that the transceiver 120 may generate its own signal indicative of the wetting event. In such other embodiments, the transceiver 120 can itself provide the signal indicative of a wetting event.

The transceiver 120 can include a signal coupling from the sensor 110 to the alert processor 130.

For one example, the transceiver 120 can include a wired node 121 coupled to the sensor 110 at a first location and to the alert processor 130 at a second location.

For another example, the transceiver 120 can include a wireless transmitter 122 receiving an alert or other signal from the sensor 110 and sending the alert or other signal 123 to the alert processor 130. As described herein, an alert or other signal 123 includes one or more of (1) an alert, that is, an indicator that a wetting event has started or has occurred, or (2) another signal, that is, an EM (electromagnetic) or other informational signal with respect to the progress of a wetting event. In this context, a signal can include a measure of an amount of wetting, encoded onto an EM carrier in AM (amplitude modulation) or another form of signal modulation.

Further, while in this Application, an alert or other signal 123 is primarily described with respect to EM carriers, there is no particular requirement for any such limitation. For example, the alert or other signal 123 can be sent using a sonic carrier or another form of sending information.

As described herein, the transceiver 120 can include a wireless converter 124. The wireless converter 124 can be coupled both to a legacy wireless receiver 125 (not part of the transceiver 120), and to the wireless transmitter 122. This can have the effect that the transceiver 120 can receive a wireless signal 123 configured for the legacy wireless receiver 125 and retransmit that wireless signal 123 to the alert processor 130.

As described herein, this can have the effect that a user of the legacy wireless receiver 125 can couple that legacy wireless receiver 125 to the wireless transmitter 122 (that is, to the wireless converter 124) without having to replace the legacy wireless receiver 125 to use the system 100.

### Alert Processor

The alert processor 130 can include elements shown in the figure. It includes at least the following: a mobile device 131 (such as a cellular telephone, music player, or similar mobile device; a "smart watch" or other wearable or implantable computing device; a portable computer such as a Raspberry Pi^{™}; or any similar device) operating under control of a mobile app 132. The mobile device 131 can include a processor 131a, memory and storage 131b, one or more input elements 131c, and one or more output elements 131d. For example, the mobile device 131 can include an Android^{™} cellular telephone available from Google, Inc. of Mountain View, California (and other suppliers), or an iPhone^{™} cellular telephone available from Apple Computer of Cupertino, California; including a standard processor 131a, memory and storage 131b, input elements 131c and output elements 131d (such as one or more accelerometers or gyroscopes, one or more buttons, a capacitive touch and display screen, a GPS locator, one or more short-range wireless communicators (such as Bluetooth^{™} or NFC, near-field communication), one or more speakers, one or more switches), and other equipment known to be used with such devices.

As described herein, the mobile device 131 operates under control of the mobile app 132. As described herein, the mobile app 132 can operate using a computing device, and can include instructions and data maintained in the mobile device 131, wherein the instructions can be executed or interpreted using an operating system resident on the mobile device 131. For example, the operating system can include iOS 9.3.2^{™}, or any similar operating system, and the mobile app 132 can include instructions and data suitable to perform the functions described herein.

As described herein, the mobile device 131 can receive alerts or other signals from the transceiver 120. The technique for receiving alerts or other signals can use the instructions in the mobile app 132, or can use instructions originally having a different purpose, but which are effective to receive alerts or other signals 123. For example, the mobile device 131 can receive those alerts or other signals 123 in memory or storage 121b, and maintain digitized versions thereof.

The mobile app 132, by its instructions, can: (a) analyze those alerts or other signals; (b) determine whether and when to publish alarms to recipients, such as to patients, to caregivers, to substitute caregivers, to data storage, and possibly otherwise; and can (c) present published alarms to recipients in a form that attracts the attention of those recipients.

### Do Alerts Indicate an Actual Wetting Event?

For one example, the mobile app 132 can receive alerts or other signals 123 from the transceiver 120 and determine if those alerts or other signals 123 indicate an actual wetting event, as opposed to noise or a likely false positive.

The mobile app 132 can execute instructions from the memory or storage 121b, wherein those instructions direct the mobile device 131 to compare those alerts or other signals 123 with one or more threshold values. In such cases, the mobile app 132 can determine that those alerts or other signals 123 indicate a wetting event if sufficient current passes through the electrical circuit 111 in the sensor 110 (because the wicking element 112a is sufficiently wet, because fluid has vented from the body exit 116). Accordingly, the mobile app 132 can compare incoming alerts or other signals 123 with a threshold to determine if the amount of current is sufficient.

As described herein, the threshold can be adjusted to account for the possibility that perspiration is also vented onto the wicking element 112a of the replaceable test strip 112. If the mobile app 132 notes greater amounts of perspiration, it can raise the threshold, and vice versa.

### Automatically Recording Information About a Wetting Event

For another example, the mobile app 132, having determined that received alerts or other signals represent a wetting event, can record information about the event. For example, the mobile app 132 can maintain information about the received alerts or other signals in the memory and storage 131b, or can use the one or more output elements 131d to present that information to a user, or to send that information to the server 150.

In one possible implementation, information about the alert can include one or more of the following:
A record that the alert occurred, and a date and time of the alert, such as a number of days that have passed since an earlier alert: This can be valuable to determine whether the patient is learning to sleep through the night without a wetting event, or is making progress to that goal.

A duration of the alert, such as a time from a start of the wetting event to a time when a response is made to the alert, or such as a time from when the response is made to the alert, to a time when the alert is turned off or the sensor is removed: This can be valuable to determine whether the patient is learning to respond to the alert, such as to remove the sensor and to use the restroom.

A record of improvement by the patient, such as either raw improvement, or with respect to a guide to improvement. For example, in some recommendations, it is desirable for the patient (such as a child) to sleep dry through the night (that is, no wetting events all evening) for at least 14 days to determine that the patient has broken the habit of wetting events. In such cases, the mobile app 132 can determine whether the patient has made substantial progress, and how much further progress is desired before the habit of wetting events can be declared broken.

### Altering Sensor Parameters In Response to the Patient

The mobile app 132 can also cause the alert processor 130 to direct the sensor 110 to alter its electrical or other parameters, such as by adjusting a resistance of the electrical circuit 111 using a potentiometer (coupled to the electrical circuit 111), in response to signals from the sensor 110.

In such cases, for example, if the patient perspires excessively, the alert processor 130 can reduce the sensitivity of the sensor 110 (such as by increasing the resistance of the electrical circuit 111) so that the sensor 110 does not generate a false positive for a wetting event.

In such cases, for another example, when a wetting event occurs, the mobile app 132 can also cause the alert processor 130 to direct the sensor 110 to eliminate an electrical current through the electrical circuit 111 in the sensor 110, so that the patient does not suffer burns or rash due to that current.

In such cases, for another example, when the caregiver replaces the replaceable test strip 112 after a wetting event, the mobile app 132 can also cause the alert processor 130 to direct the sensor 110 to restart the electrical current through the electrical circuit 111 in the sensor 110 in preparation for a possible future event.

The mobile app 132 can also cause the alert processor 130 to direct the sensor 110 to alter its electrical or other parameters in response to information so far collected about the patient (such as a child).

For example, the mobile app 132 can maintain a record of chemical aspects of wetting events, such as an amount of acidity or salinity. If the mobile app 132 determines that changes in chemical aspects of wetting events are significant enough to advise changes in parameters of the sensor 100, the mobile app 132 can cause the alert processor 130 to direct the sensor 110 to alter the parameters of the electrical circuit 111 (such as by using a potentiometer, as described herein, or by using another circuit element to otherwise alter the parameters of the electrical circuit 111).

For another example, the mobile app 132 can maintain a record of volumetric aspects of wetting events, such as in response to a time duration of each wetting event. Similarly, If the mobile app 132 determines that changes in chemical aspects of wetting events are significant enough to advise changes in parameters of the sensor 100, the mobile app 132 can cause the alert processor 130 to direct the sensor 110 to alter the parameters of the electrical circuit 111, such as by adjusting a timing circuit to automatically break the electrical circuit 111 after a selected duration during which the wetting event has continued.

### Recording Timing Information About a Wetting Event

The mobile app 132 can also cause the alert processor 130 to direct the sensor 110 to record and maintain information with respect to the during, timing, and other information, with respect to wetting events.

In one particular implementation, as described herein, the mobile app 132 can automatically record and maintain information (such as at the server 150) with respect to wetting events, while the patient or caregiver is possibly preoccupied with responding to those wetting events. Since the mobile app 132 can perform these actions without human intervention, this can have the effect that information with respect to wetting events can be maintained accurately, despite the possibility of human error in remembering to record those wetting events, or the possibility of human error in accurately recording, pertinent information with respect to those wetting events.

For example, the mobile app 132 can record the occurrence, duration, and other timing aspects of wetting events. As noted herein, this can include, without limitation, (1) how frequently wetting events occur, (2) an amount of time between an earlier wetting event and a current wetting event, (3) a first, second, or other derivative of a measure of times between wetting events, (4) an amount of time taken by the patient, such as a child, or a caregiver, such as a parent, in responding to the wetting event, (5) a first, second, or other derivative thereof, or another timing or other responsive measure with respect to wetting events.

For example, as described herein, the mobile app 132 can record and maintain information with respect to how much time, such as a number of days, which have passed since the most recent wetting event. This can be valuable in determining whether the patient has been free of wetting events for 14 days, after which the patient can be deemed free of any substantial likelihood of future wetting events.

Naturally, the value of the time duration of "14 days" can be adjusted in response to circumstances, such as (1) a significant, or even just a recent, history of wetting events; (2) becoming subject to or resuming significant life stressors; (3) having relapsed to wetting events after earlier having been deemed free of future wetting events; (4) becoming subject to or resuming a medical ailment, such as a bladder infection, insomnia, or other physical or psychological condition; or alternatively, (5) in response to the nature of the wetting events, such as whether those wetting events are relatively larger or smaller than earlier recorded. For example, the mobile app 132 can choose to substantially ignore wetting events below a certain volume threshold (such as only a "dribble") when determining the 14-day dryness duration.

The mobile app 132 can adopt one or more policies, such as might be set as a default by the manufacturer, or set by the patient or caregiver for a sequence of wetting events, with respect to determining when the patient is making progress with respect to wetting events. These policies can include those described above, and can include determining a selected time duration after which the patient has made sufficient progress with respect to wetting events that monitoring is no longer significantly necessary. Moreover, these policies can be adjusted by the patient or caregiver, or can be updated by the manufacturer as part of a software update.

In addition, the mobile app 132 can record and maintain information with respect to how long it takes the patient to respond to a wetting event alarm, to terminate the alarm, and to attend to the restroom in lieu of continuing the wetting event. For example, when a wetting event is detected, the alert processor can start a timer that records the amount of time it takes for the patient to respond to the wetting event alarm, such as "30 seconds to respond on Monday; 28 seconds to respond on Tuesday; 29 seconds to respond on Wednesday; no wetting event on Thursday; 27 seconds to respond on Friday;" or similar patient progress. Naturally, a shorter amount of time is likely to be considered better. The alert processor can maintain the amount of time for each wetting event, and determine whether the patient is responding more quickly (good) or failing to respond more quickly (not as good).

In addition, the mobile app 132 can receive information from the sensor with respect to movement by the patient. For example, the sensor can include one or more accelerometers, such as a digital triaxial acceleration sensor. For another example, the one or more accelerometers, such as a digital triaxial acceleration sensor can be included in the transceiver. In either technique, the one or more accelerometers can determine when the patient is moving. When the patient is merely moving as part of sleeping, the sensor will not detect a wetting event, so the patient's movement can be discounted. When the sensor detects a wetting event, the accelerometer can detect the patient's movement (such as at a selected degree of sensitivity), with the effect of determining how quickly the patient responds to the alarm. Improved time to respond to the alarm should indicate progress by the patient. Moreover, the sensor can respond to the accelerometer to turn off the alarm without action by the patient or the caregiver, to turn on the lights in the patient's bedroom, or other appropriate actions. In such cases, the sensor does not have to wait for the patient or caregiver to locate and hit a button or switch to indicate that they are responding to the wetting event, but can respond directly and allow the sensor to measure that they are making that response.

### Recording Timing Information About a Wetting Event

For another example, the mobile app 132, having determined that received alerts or other signals represent a wetting event, can determine to which recipients to publish an alarm. For example, the patient 161 can have more than one caregiver 162 associated therewith, such as: a child with a babysitter and one or more parents, or a child with two parents, or a hospital patient with a designated nurse and one or more backup nurses; each of whom is designated as a possible caregiver 162 who will attend to the child if a wetting event occurs.

In such cases, the mobile app 132 can also cause the alert processor 130 to publish one or more alerts to the one or more caregivers 162.

In such cases, for example, the mobile app 132 can publish the alert to a first caregiver 162 a selected number of times. In such examples, the mobile app 132 can publish the alert with a first degree of urgency a first time (such as sounding an alarm to indicate a wetting event), with a second degree of urgency a second time (such as sounding a more urgent alarm to indicate a wetting event that has not yet been dealt with), and possibly with third or further degrees of urgency thereafter.

In such cases, for another example, the mobile app 132 can publish the alert to one or more first caregivers 162 a first time, to one or more second caregivers 162 a second time, and possibly to third or other caregivers 162 thereafter. The mobile app 132 can also publish the alert with varying combinations of degrees of urgency, number of caregivers 162, selected caregivers 162, and otherwise to inform caregivers 162 of the fact of, and information about, the wetting event. The mobile app 132 can also publish the alert to the patient 161 themselves, with the effect that the patient 161 can hear the alarm, awake, and attend to the wetting event (and take themselves to the restroom).

### Providing Identifying Information About the Patient

The mobile app 132 is configured to receive alert signals from two or more possible patients 161 concurrently, such as for all of the patients 161 in a wing or on a floor of a hospital, or assigned to a particular nurses' station, or such as multiple small children in a children's daycare facility. The mobile app 132 can associate selected particular patients 161 with selected particular caregivers 162.

In one example embodiment, the mobile app 132 can associate particular patent 161 A with particular caregiver 162 B, and can associate particular patent 161 C with particular caregiver 162 D. The mobile app 132 can receive alerts and other signals from both patient 161 A and patient 161 C, and can publish alerts to both caregiver 162 B and caregiver 162 D.

In this example embodiment, when patient 161 A has a wetting event, the mobile app 132 can publish an alert to associated primary caregiver 162 B. If caregiver 162 B fails to attend to the wetting event, the mobile app 132 can publish a second alert to associated secondary caregiver 162 D. Similarly, when patient 161 C has a wetting event, the mobile app 132 can publish an alert to associated primary caregiver 162 D. If caregiver 162 D fails to attend to the wetting event, the mobile app 132 can publish a second alert to associated secondary caregiver 162 B.

This can have the effect that caregiver 162 B provides backup for caregiver 162 D, and vice versa. Other and further possibilities exist. For example, patient 161 A can have their own primary caregiver 162 B, patient 161 C can have their own primary caregiver 162 D, and caregiver 162 E can provide backup to more than one caregiver 162 (B and D).

In another example embodiment, the mobile app 132 can associate more than one particular patient 161, such as both particular patient 161 A and particular patient 161 F, with a primary caregiver 162 B, and possibly a secondary caregiver 162 D.

In this example embodiment, when patient 161 A has a wetting event, the mobile app 132 can publish an first type of alert to primary caregiver 162 B, such as a first sound pattern for the alarm. When patient 161 F has a wetting event, the mobile app 132 can publish a second type of alert, also to primary caregiver 162 B, such as a second sound pattern for the alarm. For example, the first sound pattern could be the song "Purple Rain," while the second sound pattern could be the song "Raindrops Keep Falling On My Head" or "Yellow Submarine."

This can have the effect that primary caregiver 162 B can determine, from the nature of the sound pattern for the alarm, which particular patient 161 (A or F) has had the wetting event. In the context of the invention, there is no particular requirement that the sound alarm for the patient 161 and the caregiver 162 are the same; however, it might be convenient to make those sound alarms the same so the caregiver 162 is not confused by two different and concurrent sound alarms.

This can have the effect that the primary caregiver 162 B can attend to wetting events for multiple patients 161 (A and F), so long as those patients 161 do not have their wetting events concurrently. In such cases, the mobile app 132 can determine that more than one wetting event is occurring concurrently, and immediately publishes an alert to the secondary caregiver 162 D without waiting for the primary caregiver B to fail to respond timely.

Each patient 161 can have more than one caregiver 162 associated therewith. The alert processor 130 can alert those caregivers 162 in series or in parallel.

For an example of alerting caregivers 162 in series, the alert processor 130 can fail-over from one caregiver 162 to another (such as, B to D to E, to possibly another set of caregivers) in order, when it determines that any particular caregiver 162, or set thereof, do not attend to the patient 161. In such cases, the alert processor 130 can also reorder those caregivers 162, either because selected ones are more attentive or less attentive, or because selected ones are known to be currently attending to a different patient 161.

For an example of alerting caregivers 162 in parallel, the alert processor 130 can publish the alert to more than one caregiver 162 (such as, B and D and E, and possibly other caregivers) concurrently, canceling the alert when one of those caregivers 162 (such as B) indicates they are attending to the patient 161. The alert processor 130 can obtain a message from the alert presenter 140 that one of the caregivers 162 B has noted the alert, or can obtain a message from the alert presenter 140 that one of the caregivers 162 B is physically moving toward, or physically near, the patient 161.

More generally, the alert processor 130 can associate groups of patients 161 with groups of caregivers 162, alerting whichever ones of those caregivers 162 it chooses when it detects a wetting event for any one of those patients 161. The alert processor 130 can also organize groups of caregivers 162 into subgroups, alerting caregivers 162 in those subgroups in parallel or series, and alerting those subgroups themselves in parallel or series. For example, the alert processor 130 can select a group of primary caregivers 162, alerting them in parallel or series as it chooses, a group of secondary caregivers 162, alerting them in parallel or series as it chooses when the primary caregivers 162 do not tend to the patient 161, a group of tertiary caregivers 162, and so on.

This can have the effect that the mobile app 132 can connect patients to caregivers in an ***m* : *n*** multiplicity rather than a ***1* : *1*** multiplicity.

### Measuring Response Information About the Wetting Event

For another example, the mobile app 132, having determined to which recipients to publish alarms, can receive information from those recipients with respect to whether those recipients respond to the alarms. In this context, if a selected recipient does not respond, the mobile app 132 can publish the alarm to a substitute recipient. This can include a cascade of substitute recipients:
If the alert processor 130 publishes an alarm directly to the patient (such as using a loud sonic alarm), without the patient responding, the mobile app 132 can fail-over to publishing the alarm to a primary caregiver.

If the alert processor 130 publishes an alarm to the primary caregiver for that patient (such as using either a sonic alarm or a flashing screen), without that caregiver responding, the alert processor 130 can fail-over to publishing the alarm to a substitute caregiver.

If the alert processor 130 publishes an alarm to the substitute caregiver (again, such as using either a sonic alarm or a flashing screen), the alert processor 130 can fail-over to publishing the alarm to another caregiver.

The alert processor 130 can also publish the alarm to a data storage element, such as the memory and storage 131b for the mobile device 131, or such as a database (not shown in this figure) coupled to the mobile device 131, both as a record that the alarm was published, and as a message for another recipient to review the published alarm.

As described herein, when the alert processor 130 publishes an alarm to a selected recipient, it sends that alarm to the alert presenter 140 associated with that selected recipient. The alert presenter 140 presents the alarm to the selected recipient, determines if the selected recipient responds to the alarm, and so informs the alert processor 130. If the alert presenter 140 informs the alert processor 130 that the selected recipient is not responding to the alarm, the alert processor 130 can fail-over to the next selected recipient.

While the mobile device 131 is primarily described with respect to a cellular telephone, the alert processor 130 can instead include another type of general-purpose processor, or a type of special-purpose processor specifically configured for the purposes described herein, or other electronic circuitry configured for the purposes described herein.

### Alert Presenter

The alert presenters 140 each can include elements shown in the figure, such as at least the following: a mobile device similar to the alert processor 130. For example, the alert presenters 140 can include an iPhone 6^{™} cellular telephone similar to the alert processor 130. For another example, the alert presenters 140 can include another device, such as a computing device (desktop, laptop, wearable or implantable), or other device capable of receiving signals and alerting the user.

The alert presenter 140 can be associated with the alarm recipient, and can receive the published alarm from the alert processor 130. As described herein, when the alert processor 130 determines the presence of a wetting event, the alert processor 130 publishes an alarm to one or more selected recipients.

If the alert presenter 140 publishes an alarm directly to the patient, it can provide a loud sonic alarm to wake the patient quickly. In one alternative, if the patient does not wish to be awakened by a sonic alarm, the alert presenter 140 can publish the alarm to the patient by flashing a light. In another alternative, the alert presenter 140 can publish the alarm to the patient by vibrating or generating another type of haptic, or other, alert.

If the alert presenter 140 publishes an alarm to a caregiver for that patient (such as either the primary caregiver or a substitute caregiver), it can provide one or more of: a sonic alarm or a flashing screen, such as on a cellular telephone for the caregiver.

If the alert presenter 140 publishes an alarm to a data storage element, such as data storage for a cellular telephone, or for the server 150, or such as a database (possibly available at the remote element 155 of the server 150), the alert presenter 140 can send a message including information that the alarm was published, and can send a message for another recipient to review the published alarm.

### Server

The server 150 can include elements shown in the figure, such as at least the following: a computing device (including a processor 151, memory or storage 152), a communication link 153, an input/output port 154), and other elements necessary or sufficient for the functions described herein. The server 150 can also couple to one or more remote elements 155 (which are not actually part of the system 110 or the server 150).

The server 150 can couple to the alert processor 130 or the alert presenter 140, such as using the communication link 153 and the input/output port 154. For one example, the communication link 153 can include a telephone switching system and the input/output port 154 can include a telephone number. For another example, the communication link 153 can include a computer network (such as the Internet) and the input/output port 154 can include a network interface card.

In such cases, the server 150 can receive messages from either the alert processor 130 or the alert presenter 140. The messages can include data with respect to patients, caregivers, wetting events, and other information related to patient care or customer relations. The server 150 can send messages to the alert processor 130 or the alert presenter 140.

In such cases, receiving or sending messages with the alert processor 130 or the alert presenter 140 has the effect of allowing the server 150 to communicating with the patient, caregivers, or alert system customer. This allows the server 150 to perform one or more of the following functions:
The server 150 can collect information with respect to wetting events. The server 150 can forward this information to the patient, to caregivers (such as to substitute caregivers or persons in supervisory positions with respect to caregivers), or to alert system customers.

The server 150 can collect information with respect to patients. For example, the server 150 can determine whether the patient (or particular patients, when there is more than one patient coupled to the alert system 100) is successfully learning to avoid wetting events. It is sometimes asserted that patients should be free of wetting events for about 14 days to assure that enuresis is deemed "cured." In such cases, the server 150, or the alert processor 130, can determine, in response to information about wetting events, how many more days the patient should remain free of wetting events before their enuresis is deemed cured.

The server 150 can collect information with respect to caregivers. For example, the server 150, or the alert processor 130, can determine an average response time for particular caregivers, or a number of times each particular caregiver has been alerted to care for a patient. In the former such case, the server 150, or the alert processor 130, can provide feedback to the caregiver. In the latter such case, the server 150, or the alert processor 130, can determine a next caregiver to present an alert or other signal to, in response to an attempt to balance caregiving load.

The server 150, or the alert processor 130, can collect information with respect to sensors 110. For example, when wetting events occur, and the patient or caregiver reports this, but the sensor 110 failed to report this, the server 150, or the alert processor 130, can adjust parameters with respect to the sensor 110 (such as sensitivity to moisture, loudness of alarm, or other parameters related to detecting wetting events or presenting alarms), or can inform a customer of the alert system 100 that the sensor 110 should be replaced.

The sensor 150 can collect backup information in the event that the alert processor 130 is damaged, loses memory, or is otherwise disabled. For example, when the alert processor 130 is replaced or upgraded, stored information for use by the alert processor 130 can be restored from backup information maintained at the server 150.

The sensor 150, or the alert processor 130, can collect information with respect to usage of the alert system 100. For one example, the sensor 150, or the alert processor 130, can inform the patient, caregiver, or customer of the alert system 100 that more (or fewer) alert processors 130 or alert presenters 140 should be integrated with the alert system 100. For another example, the sensor 150, or the alert processor 130, can inform the patient, caregiver, or customer of the alert system 100 that the patient has learned to avoid wetting events, and usage of the alert system 100 can be stopped.

The sensor 150, or the alert processor 130, can collect business information with respect to interaction between the alert system 100 and its supplier. For one example, depending on the nature of the business arrangement between the supplier and the customer, the sensor, or the alert processor 130, can collect information related to billing, repairs, quality assurance, and other information assisting the supplier and customer in obtaining value received from the alert system 100. For another example, the server 150 can collect information related to system components and type, repair and help-desk records, and successful (or unsuccessful) solutions, to assist the customer in their experience with requests for service of the alert system 100.

In one or more embodiments, the server 150 can be coupled to one or more remote elements 155. In this context, the terms and phrases "remote," "remote element," and variants thereof, refer to logical remoteness, not necessarily to physical remoteness or to difficulty of connectivity. For example, remote elements 155 can be coupled to the server 150 by sharing the memory or storage 152 with the server 150, by sending and receiving messages to and from the server 150, by coupling to the input/output port 154, or by any other technique allowing information to flow at least from the server 150 to a remote element 155 or to the server from a remote element 155.

For example, remote elements 155 can include one or more of the following: a remote memory or storage, such as for archive or backup, cloud storage, or memory or storage for use by another device; a remote processor, such as a special-purpose processor, a processor operating under control of special-purpose software, a particular electrical circuit, a cloud computing device, or any other computing element not incorporated into the system 100; or any other device not particularly specified herein, but useful with the system 100.

### Patient, Caregivers, Operators

As described herein, the patient 161 can include a child, such as a child who has difficulty sleeping dry through the evening, or otherwise having wetting events, an elderly person with similar problems, a hospital or other medical patient with similar problems, a patient with one or more leaking bodily orifices, such as a bleeding or suppurating wound, or another type of person subject to wetting events. In alternative embodiments, a "patient" 161 can be sufficiently general to include a device, sensor, or other equipment subject to wetting events, such as the possibility of leakage from a canister, or a similar concern.

As described herein, the caregivers 162 can include one or more of: a parent, a nurse, medical personnel, or other persons charged with the responsibility for noting wetting events and responding thereto. For example, caregivers can include a babysitter or one or both parents for a child, a patient's assigned nurse and one or more backup nurses, an attending physician and one or more on-call physicians, or any other person who can reasonably be expected to respond to an alert or other signal.

As described herein, the operators 163 can include any person who can reasonably be expected to respond to alerts or other signals from the system 100.

### Server Interface

The server interface 170 can include elements shown in the figure, such as at least the following: a mobile device similar to the alert processor 130. For example, the server interface 170 can include an iPhone 6^{™} cellular telephone similar to the alert processor 130. For another example, the server interface 170 can include another device, such as a computing device (desktop, laptop, wearable or implantable), or other device capable of receiving signals and alerting the user.

The server interface 170 can be associated with a user of the system 110, such as an operator 163, and can interact with the server 150 to retrieve information with respect to wetting events, alerts, history for patients 161, history for caregivers 162, history for elements of the system 110, and any business interactions between the user and a provider of the system 110.

### 5.2.2. Figure 2

Figure 2 shows a conceptual drawing of a clamp.

A clamp 112f and the waterproof box 115 can collectively include a plastic (or otherwise urine-resistant, or resistant to the fluid to be detected) box-like object, as shown in the figure. As described herein, these are collectively referred to as a clamp assembly 200.

The clamp assembly 200 can be coupled to the replaceable test strip 112, as shown in the figure, or in some other similar configuration. As otherwise and further described herein, the clamp assembly 200 can include elements shown in the figure, including without limitation: a clamp base 201, a test strip receiving zone 202, an attachment point 203, a cover 204, one or more visible indicators 205, and possibly other elements, or possibly other aspects of those elements described herein.

The clamp base 201 can include a substantially box-like object, as shown in the figure, with substantially rounded sides and beveled edges. This can have the effect of limiting the likelihood of cuts or bruises to the patient when the clamp base 201 is disposed near the body exit 116 or otherwise within the patient's clothing.

However, in the context of the invention, there is no requirement for any such limitation. For example, the clamp base 201 can include a substantially rectilinear box-line object, possibly with rounded corners and possibly with beveled edges, but not required. Similarly, the clamp base 201 can include a substantially ellipsoidal object, possibly with attachments and possibly with jutting elements, but not required. The clamp base 201 need only be able to perform the functions described herein.

The test strip receiving zone 202 can include a substantially flat region bounded by a bottom 202a (defined by a portion of the clamp base 201), a top 202b (defined by a portion of the cover 204), and disposed in size and shape to receive one or more replaceable test strips 112.

The attachment point 203 can be disposed substantially in or near the test strip receiving zone 202, and can include a protrusion onto which the replaceable test strip 112 can be attached. For example, the replaceable test strip 112 can be punctured by the protrusion, with the effect of anchoring the replaceable test strip 112 within the receiving zone 202.

However, in the context of the invention, there is no requirement for any such limitation. For example, the attachment point 203 can include a substantially crimping region, such as possibly an edge of the cover 204, disposed suitably for holding the replaceable test strip 112. For another example, the attachment point 203 can include a substantial detent, into which the replaceable test strip 112 can be fitted. The attachment point 203 need only be able to perform the functions described herein.

The cover 204 can include a hinge 204a coupled to one or more points on the clamp base 201, the hinge 204a being capable of providing the cover with at least two possible positions: open and closed. In the open position, the replaceable test strip 112 can be placed substantially in or near the test strip receiving zone 202, and can be coupled to the attachment point 203. In the closed position, the replaceable test strip 112 can be held at least partially within or near the test strip receiving zone 202. Other possible positions are possible, such as possibly a partially-open partially-closed position.

The visible indicators 205 can be disposed to provide a visible indication of when the clamp base 201 is open, closed, or in some other state. For example, the visible indicators 205 can include liquid crystal diodes (LCD's), light-emitting diodes (LED's), incandescent lamps, or other devices that can be seen by a user, particularly a casual user who is not making a close inspection, but is just looking at the visible indicators 205 to see its status.

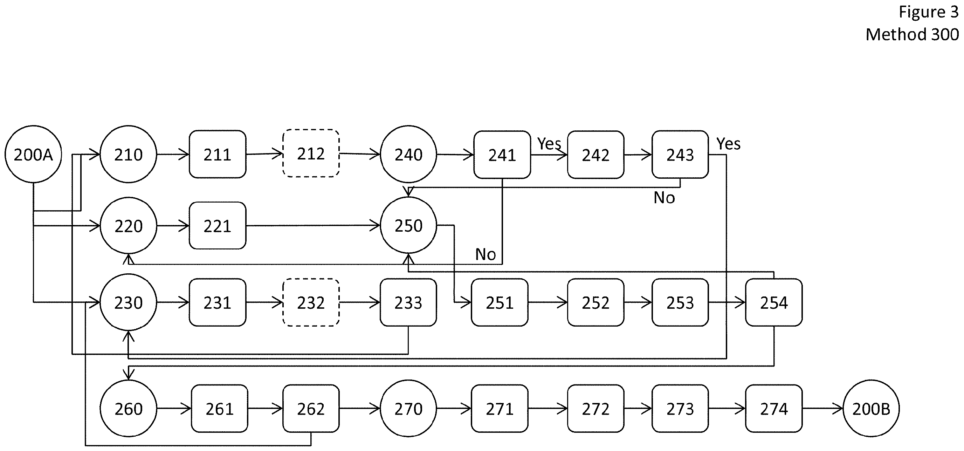

### 5.2.3. Figure 3

Figure 3 shows a conceptual drawing of a method.

A method 300 of using the system 100 can include flow points and process steps shown in the figure, such as at least the following:
at a flow point 310, a portion of the method relating to disposing the sensor 110 to detect wetting events, and an (optional) portion of the method relating to disposing the transceiver 120 to send signals with respect to wetting events;
at a flow point 320, a portion of the method relating to disposing the sensor 110 to detect, and the transceiver 120 to send, signals with respect to wetting events;
and a flow point 330, a portion of the method relating to (re-)disposing the sensor 110 to detect, and the transceiver 120 to send, signals with respect to wetting events;
at a flow point 340, a portion of the method relating to, by the alert processor 130, receiving and processing signals with respect to wetting events;
at a flow point 350, a portion of the method relating to, by the alert processor 130, publishing alerts to the alert presenters 140; and
at a flow point 360, a portion of the method relating to, by the alert processor 130, publishing alerts to the server 150;
at a flow point 370, a portion of the method relating to, by a server interface 170, interacting with the server 150.

A flow point 300A indicates the method 300 is ready to start.

### Disposing the sensor

A flow point 310 indicates the method 300 is ready to dispose the sensor 110 to detect a wetting event.

At a step 311, the caregiver 162 (or the patient 161) disposes the replaceable test strip 112 in the clamp 113, and disposes the clamp 113 near the body exit 116.

At an optional step 312, the caregiver 162 couples the legacy wireless receiver 125 to the wireless converter 124 and to the wireless transmitter 122. This can have the effect that the legacy wireless receiver 125 can receive the signal 123 from the sensor 110 and retransmit that signal 123 to the alert processor 130.

The method 300 proceeds with the flow point 340, at which the method 300 is ready to receive and process signals, unless and until a wetting event occurs.

### Wetting Event Occurs

At a flow point 320, a wetting event occurs.

At a step 321, the replaceable test strip 112 receives fluid from near the body exit 116, as a consequence of a wetting event. The wicking element 112a of the replaceable test strip 112 draws the fluid, causing the first conductive node 112b to couple to the second conductive node 112c. This can cause the positive node 111b to couple to the negative node 111c, which can complete the electrical circuit 111, which can provide the signal 123 at the output node 114 of the sensor 110. This can cause the transceiver 120 to send the signal 123 to the alert processor 130.

The method 300 proceeds with the flow point 350, at which the method 300 publishes alerts to caregivers 162, unless and until a caregiver 162 attends to the patient 161.

### Wetting Event Tended To

At a flow point 330, the wetting event has been tended to.

At a step 331, the caregiver 162 (or the patient 161) removes the old replaceable test strip 112 from the clamp 113, and discards the old replaceable test strip 112.

At a step 332, if the wetting event is still ongoing, the patient 161 continues urination at a restroom. The caregiver 162 wakes the patient 161 to perform this step if necessary or desirable.

At a step 333, once the patient 161 completes their tasks at the restroom, the method 300 proceeds with the flow point 310, at which the sensor 110 is redis-posed for a possible next wetting event by disposing a new replaceable test strip 112.

### Receiving and processing signals

A flow point 340 indicates that the method 300 is ready to receive and process signals with respect to wetting events.

At a step 341, the alert processor 130 receives the signal 123 from the transceiver 120. The alert processor 130 provides the signal 123 to the mobile app 132, which determines whether the signal 123 indicates a wetting event. If not, the method 300 returns to the flow point 320. If so, the method 300 proceeds with the next step.

At a step 342, the alert processor 130 publishes an alert to the patient 161.

At a step 343, the alert processor 130 determines if the alert to the patient 161 is sufficient to deal with the wetting event. If so, the method 300 proceeds with the flow point 330, at which the patient 161 tends to the wetting event. If not, the method 300 proceeds with the flow point 350, at which the alert is published to one or more caregivers 162.

### Publishing alerts to caregivers

A flow point 350 indicates that the method 300 is ready to publish alerts with respect to wetting events to caregivers 162.

At a step 351, the alert processor 130 selects a set of one or more caregivers 162 to whom to publish the alert. As described herein, this set of caregivers 162 can be statically associated with the patient 161, or can be selected dynamically with the patient 161 by the alert processor 130.

At a step 352, the alert processor 130 publishes the alert to the set of selected caregivers 162. As described herein, publishing the alert to the set of selected caregivers 162 can include sending a message from the alert processor 130 to the one or more alert presenters 140 associated with the selected caregivers 162. For example, when the alert processor 130 and the alert presenters 140 are each mobile devices, such as cellular telephones, the alert processor 130 can send a text message, voice message, or other data message, to the alert presenters 140 to that effect.

The method 300 proceeds, as described herein with respect to the flow point 360, with presenting the alert to the selected caregivers 162. When the alert presenter 140 succeeds in causing at least one of the selected caregivers 162 to attend to the patient 161, the alert presenter 140 sends a message back to the alert processor 130 to that effect. Similarly to sending a message from the alert processor 130 to the alert presenter 140, the alert presenter 140 can send a text message, voice message, or other data message, to the alert processor 130 to that effect.

If the alert processor 130 receives a message from the alert presenter 140 that one of the selected caregivers 162 is tending to the patient 161, the method 300 proceeds with the next step 353. If not, the method 300 skips the next step 353 and proceeds with the step 354 thereafter.

At a step 353, the alert processor 130 receives the message from the alert presenter 140 that one of the selected caregivers 162 is attending to the patient 161. The method 300 proceeds with the flow point 330, at which the method 300 redisposes the sensor 110 by discarding the old replaceable test strip 112 and disposing a new replaceable test strip 112.

At a step 354, the alert processor 130 receives the message from the alert presenter 140 that one of the selected caregivers 162 is attending to the patient 161. The method 300 proceeds with the flow point 350, at which the method 300 selects a new set of one or more caregivers 162 to attend to the wetting event, and publishes the alert to this new set of caregivers 162.

The method 300 repeats the step 353 and the step 354 until at least one caregiver 162 tends to the wetting event.

### Operation of the alert presenter

A flow point 360 indicates that the method 300 is ready to present alerts with respect to wetting events.

At a step 361, those alert presenters 140 selected by the alert processor 130 present their alerts to their associated caregivers 162. For example, when alert presenters 340 include cellular telephones, the alert presenters 140 can buzz, ring, or flash, to tell caregivers 162 to attend to a wetting event.

At a step 362, each alert presenter 140 determines whether its associated caregivers 162 is attending to the patient 161, or moving to attend to the patient 161. The alert presenter 140 can
(1) receive a signal from its associated caregiver 162, such as by having the caregiver 162 tap a button on the alert presenter 140 or speak a code phrase into the alert presenter 140;
(2) determine if its associated caregiver 162 is moving toward the patient 161, such as by using an accelerometer or positioning system, the latter possibly including a GPS locator system;
(3) determine if its associated caregiver 162 is near enough to the patient 161 to attend to the wetting event, such as by using a positioning system, such as a GPS locator system, or such as by determining a signal strength between the patient 161 and the caregiver 162;
(4) receive a signal from the patient 161 that the caregiver 162 has arrived to tend to the wetting event; or
(5) another technique by which the alert presenter 140 can determine whether its associated caregiver 162 is going to tend to the wetting event.

If the associated caregiver 162 tends to the wetting event, the method 300 proceeds with the flow point 330, at which the method 300 removes the old replaceable test strip 112 and disposes a new replaceable test strip 112 at the sensor 110. The method 300 repeats from the flow point 330 as long as the patient 161 is still at risk for wetting events.

### Interacting with the server

A flow point 370 indicates that the method 300 is ready to interact between the user and the server.

At a step 371, an operator 163 associated with a server interface 170 begins operation of the server interface 170. The server interface 170 presents information to, and receives information from, the operator 163, in response to input from the operator 163.

At a step 372, the operator 163 decides to interact with the server 150 using the server interface 170. The server interface 170 sends a message to the server 150 telling the server 150 that the operator 163 desires to interact with it.

At a step 373, the server 150 and the server interface 170 present a login screen to the operator 163, receive login information from the operator 163, and verify the login.

At a step 374, the server 150 and the server interface 170 collectively present information to, and receive information from, the operator 163, in response to input from the operator 163.

For example, the server 150 and the operator 163 can interact in one or more of the following ways:
The operator 163 can obtain detailed information about configuration of the system 100 from the server 150. The server 150 can obtain this information from its records of the user's system 100, or from diagnostics or telemetry with respect to the system 100.

The operator 163 can inform the server 150 of errors or failures of operation by the system 100. The server 150 can provide troubleshooting information, or can generate a trouble ticket for repair or replacement of one or more of the system's elements.

The operator 163 can obtain detailed information about a business relationship between the user and a provider of the system 100. For example, even though the replaceable test strips 112 are disposable, the user can rent the system 100 from the provider. For another example, the user can pay the provider for each wetting event, or for each use of the alert processor 130 or the alert presenter 140 (specifically, the mobile apps 132 associated therewith). For another example, the user can pay a one-time fee for a reasonable amount of time to train the patient 161 to avoid wetting events.

A flow point 300B indicates the method 300 is finished. The method 300 can be restarted at the flow point 300A.

### 5.3. ALTERNATIVE EMBODIMENTS AND USES

### 5.3.1. Specification Exceeds Explicit Teachings

After reading this Application, those skilled in the art will recognize that this Application includes additional teaching beyond those explicitly shown or suggested.
(1) Individual elements or method steps of the described embodiments could be replaced with substitutes that perform similar functions in other contexts.
(2) Elements of the system are described herein with respect to one or more possible perform similar functions. Moreover, as described herein, many individual elements of the described apparatuses are optional, and are not required for operation.

Moreover, although control elements of the one or more described apparatuses include more than one computing device (or more than one specialized computing device), not necessarily all similar, on which the element's functions are performed.

### 5.3.2. Similar Elements or Steps

Individual elements or method steps of the described embodiments could be replaced with substitutes that perform similar functions in other contexts.

Elements of the system are described herein with respect to one or more possible embodiments, and are not intended to be limiting in any way. In the context of the invention, there is the particular requirement for any such limitations as described with respect to any elements of the system. For example, individual elements of the described apparatuses could be replaced with substitutes that perform similar functions. Moreover, as described herein, many individual elements of the described apparatuses are optional, and are not required for operation.

Although control elements of the one or more described apparatuses are described herein as being executed as if on a single computing device, in the context of the invention, there is no particular requirement for any such limitation. For example, the control elements of the one or more described apparatuses can include more than one computing device, not necessarily all identical, on which the element's functions are performed.

Certain aspects of the embodiments described in the present disclosure may be provided as a computer program product. The computer program product can include instructions that are maintained on a computer-readable storage medium or a non-transitory machine-readable medium. A computing device can interpret the instructions and perform one or more methods as described herein. The non- transitory medium can include a magnetic, optical or magneto-optical storage medium; a flash storage medium; and/or otherwise.

### 5.3.3. Claims Included by Reference

The Claims in this Application are hereby included by reference in the text of the Specification.

### 5.3.4. Real-World Nature

After reading this Application, those skilled in the art would recognize that this Application describes techniques (including machines, methods, articles of manufacture, and compositions of matter) that can make use of new concepts to provide useful and tangible results, which provide significantly more than a software concept. In one embodiment, this Application describes techniques that can significantly improve the technologies of clean and rapid detection of wetting events, alerting and response to wetting events, and recording and processing of wetting events. Moreover, in additional embodiments, this Application describes techniques that provide meaningful limitations beyond generally linking an patent-ineligible subject matter to use of a generalized computing environment. Some of these meaningful limitations are shown herein with respect to the described embodiments.

While some embodiments are generally described herein with respect to specific steps to be performed by generalized computing devices, in the context of the invention, there is no particular requirement for any such limitation. For example, subject matter embodying the invention can include special-purpose devices; can include hardware devices having the elements described herein, and having the effect of performing the steps described herein; and combinations and/or conjunctions thereof. Embodiments of the invention are not necessarily limited to computing devices, but can also include any form of device or method that can improve techniques for improving the effect of the machine operations described herein.

## Claims

1. Apparatus including
an alert processor including a mobile device operating under control of a mobile app, the alert processor having an input port disposed to receive a signal output from a sensor in response to a fluid exiting a body of a patient, having instructions determining whether the signal output from the sensor indicates a wetting event, and having an output port disposed to send an alert signal in response to the wetting event, wherein the mobile app is configured to receive alert signals from two or more patients concurrently;
an alert presenter including an alarm responsive to the alert signal;
wherein the alert processor has access to second instructions, the second instructions directing the alert signal to at least one or more of a set of alert presenters, wherein the set of alert presenters are associated with at least two distinct caregivers;
wherein the two distinct caregivers comprise a primary caregiver and a secondary caregiver, and wherein the mobile app is configured to associate more than one patient with the primary caregiver, and
wherein the mobile app is configured to direct the alert signal to the primary caregiver, and
wherein, if the mobile app determines that more than one wetting event is occurring concurrently, the mobile app is configured to immediately direct the alert signal to the secondary caregiver without waiting for the primary caregiver to respond.

## Patentansprüche

1. Vorrichtung, die Folgendes einschließt:
einen Warnungsprozessor, der ein mobiles Gerät einschließt, das unter der Steuerung einer mobilen Anwendung arbeitet, der Warnungsprozessor aufweisend einen Eingangsanschluss, der angeordnet ist, um ein Signal zu empfangen, das von einem Sensor als Reaktion darauf ausgegeben wird, dass ein Fluid aus einem Körper eines Patienten austritt, aufweisend Anweisungen, die feststellen, ob das von dem Sensor ausgegebene Signal ein Einnässereignis anzeigt, und aufweisend einen Ausgangsanschluss, der angeordnet ist, um als Reaktion auf das Einnässereignis ein Warnsignal zu senden, wobei die mobile Anwendung dafür konfiguriert ist, Warnsignale gleichzeitig von zwei oder mehr Patienten zu empfangen,
einen Warnungsanzeiger, der eine Alarmvorrichtung einschließt, die auf das Warnsignal anspricht,
wobei der Warnungsprozessor Zugriff auf zweite Anweisungen hat, wobei die zweiten Anweisungen das Warnsignal zu mindestens einem oder mehreren eines Satzes von Warnungsanzeigern leitet, wobei der Satz von Warnungsanzeigern mit mindestens zwei unterschiedlichen Pflegekräften verknüpft ist,
wobei die zwei unterschiedlichen Pflegekräfteeine primäre Pflegekraft und eine sekundäre Pflegekraft umfassen und wobei die mobile Anwendung dafür konfiguriert ist, mehr als einen Patienten mit der primären Pflegekraft zu verknüpfen, und
wobei die mobile Anwendung dafür konfiguriert ist, das Warnsignal zu der primären Pflegekraft zu leiten, und
wobei, falls die mobile Anwendung feststellt, dass mehr als ein Einnässereignis gleichzeitig auftritt, die mobile Anwendung dafür konfiguriert ist, das Warnsignal unverzüglich zu der sekundären Pflegekraft zu leiten, ohne darauf zu warten, dass die primäre Pflegkraft antwortet.

## Revendications

1. Dispositif incluant :
un processeur d'alerte incluant un dispositif mobile fonctionnant sous la commande d'une application mobile, le processeur d'alerte comportant un port d'entrée disposé pour recevoir un signal émis en sortie depuis un capteur en réponse au fait qu'un fluide émane du corps d'un patient, comportant des instructions déterminant si le signal émis en sortie depuis le capteur indique ou non un événement de mouillage, et comportant un port de sortie disposé pour envoyer un signal d'alerte en réponse à l'événement de mouillage, dans lequel l'application mobile est configurée pour recevoir des signaux d'alerte provenant concurremment de deux patients ou plus ;
un moyen de présentation d'alerte incluant une alarme déclenchée en réponse au signal d'alerte ;
dans lequel le processeur d'alerte a accès à des secondes instructions, les secondes instructions dirigeant le signal d'alerte vers au moins un ou plusieurs moyens parmi un ensemble de moyens de présentation d'alerte, dans lequel l'ensemble de moyens de présentation d'alerte est associé à au moins deux soignants distincts,
dans lequel les deux soignants distincts comprennent un soignant primaire et un soignant secondaire, et dans lequel l'application mobile est configurée pour associer plus d'un patient au soignant primaire, et
dans lequel l'application mobile est configurée pour diriger le signal d'alerte vers le soignant primaire, et
dans lequel, si l'application mobile détermine que plus d'un événement de mouillage survient concurremment, l'application mobile est configurée pour diriger immédiatement le signal d'alerte sur le soignant secondaire sans attendre la réponse du soignant primaire.
